# EUROPEAN PATENT SPECIFICATION

(11) **EP 0 746 311 B1**
(45) Date of publication and mention of the grant of the patent: **11.07.2001**
(21) Application number: 95941909.4
(22) Date of filing: 23.12.1995
(51) Int. Cl.: A61K 9/70, A61K 47/10

(54) **MEDICINAL PATCHES FOR PERCUTANEOUS ADMINISTRATION**
MEDIZINISCHE PFLASTER ZUR PERKUTANEN VERABREICHUNG
TIMBRES MEDICAUX POUR ADMINISTRATION PERCUTANEE

(30) Priority: 24.12.1994 KR 3600654; 21.12.1995 KR 5300707
(43) Date of publication of application: 11.12.1996
(73) Proprietor: PACIFIC CORPORATION, Seoul 140-777 (KR); PACIFIC PHARMACEUTICAL CO. LTD., Seoul 140-777 (KR)
(72) Inventor: KIM, Jung Ju, Kangnam-ku, Seoul 135-240 (KR); BAE, Woo Hyun, Kangdong-ku, Seoul 134-021 (KR); LEE, Yoo Chul, Kyunggi-do 430-030 (KR); CHOI, Yang Gyu, Pacific R & D Center Dormitory, Youngin-kun, Kyunggi-do 449-900 (KR)
(74) Representative: Marchi, Massimo, Dr.
(86) International application number: KR9500171
(87) International publication number: WO9619976

(56) References cited:
- EP-A- 0 328 806
- EP-A- 0 622 075

## Description

### BACKGROUND OF THE INVENTION

### 1. FIELD OF THE INVENTION

The present invention relates to a medicinal patch which continuously delivers a certain amount of oil-soluble drugs through the skin or mucous membranes. More specifically, the present invention relates to a transdermal skin patch a multilayer laminate having 2 or more active adhesive layers, each of said adhesive layers comprising a drug, and an adhesive resin, wherein said adhesive layer contains no water and has a different water absorption capacity, and essentially contains hexylene glycol as a permeation enhancer.

### 2. DESCRIPTION OF THE PRIOR ART

Recent developments of drug delivery system, particularly a percutaneous administration system contributes to the development of pharmaceutical industry fields. The principal goal of developing drug delivery system is to improve efficiency of drug administration, safety and easiness to handle. The method of percutaneous administration offers many advantages over the traditional oral administration of drugs. For example, for the case of oral administration, a great level of drug is degraded by the metabolism in the liver prior to exhibiting its efficacy at the target site ("First-Pass Effect"). Further, oral administration has another problems such as inconsistent absorption within living body, and a frequent administration for drugs with a short life time. Besides, for the case of parenteral administration, for example injection, drawbacks such as pain at the injection site or a possible contamination has been reported. To avoid these problems associated with oral or parenteral administrations, transdermal delivery system has been actively studied and greatly developed. Representative examples of drugs of transdermal delivery system may include nitroglycerin, estradiol, scopolamine, clonidin, and nicotine.

Transdermal delivery system has advantages that it's use is easy, the active drug can be consistently absorbed into living body, and it's use can be stopped as soon as any adverse effect is noticed. However, it also has drawback. That is to say, the sufficient amount of active ingredient or drug cannot be delivered easily through skin.

Therefore, it was proposed to increase the concentration of an active ingredient contained in an adhesive layer of transdermal delivery system in order to penetrate a predetermined amount of active ingredient for a predetermined period of time to attain a desired pharmacological activity in the living body. However, this attempts has a limitation, because the human skin serves as a barrier to the penetration of active ingredient as well as to the invasion of pathogens and toxic materials, and the penetration of active ingredient is a rate-limiting step.

Another approach to enhance penetration of active ingredient is to increase the surface area of delivery system to be contact with skin. However, this approach also has disadvantages that the transdermal preparations in the form of an emulsion, ointment or cream spoil the clothes, or the preparations in the form of patch cause an increase in the production cost for the increase of size, and a inconvenient moving of patched area or a poor adherence of the patch to the skin.

Further, for the transdermal delivery system, it may cause side effects such as erythema or edema at the applied site of the skin. This becomes more serious problem for patients with chronic and frequently occurred diseases. For this case, a primary irritation and sensitization by drugs act as a dominant limiting factor for the application of transdermal delivery systems. In fact, about 20 - 30% of commercially available products show side effects on the skin.

EP-A-0 622 075 discloses tramsdermal skin patches comprising a backing layer; a liner; and between the two, a multilayer laminated of several adhesive layers, each of said adhesive layers comprising an active ingredient, an adhesive resin and a penetration enhancer (e.g. example 29). In the patches of EP-A-0 622 075 each adhesive layer has not different water absorption capacity.

To avoid such problems associated with conventional transdermal delivery systems, a miniaturization of the system, an increase of flexibility of the system, and
an enhancement of absorption of drug into the skin are required.

Under these circumstances, the present applicant developed an improved structure of medicinal patches for percutaneous administration of drugs. The structure is appropriate for transdermal delivery of oil-soluble drugs, sustain its activity for a prolonged period of time, and show a reduced irritation to the skin and improved adherence of the patch to the skin. A prior transdermal skin patch comprises a water-impermeable backing layer; a liner; and between the two, a multilayer laminated of from 2 to 5 adhesive layer, each of said adhesive layers comprising an oil-soluble drug, an adhesive resin, and a penetration enhancer, wherein each adhesive layer contains no water and has a different water absorption capacity in which the lowest layer which is to be contacted with the skin has the lowest water absorption capacity and the most upper layer in contact with the backing layer has the highest water absorption capacity.

By constituting an adhesive layer of the patch, which contains a drug, in the form of multilayer laminate, each layer having a different water absorption capacity, when the drug contained in the lowest layer in contact with the skin is penetrated into skin, the drug contained in the upper layer migrates to the lowest layer while water and watersoluble materials evaporated from the skin migrate into the upper layer, thereby the level of the drug in the lowest layer can be controlled constantly.

Therefore, a release of consistent amount of drug for a prolonged period of time can be accomplished. And, the moisture evaporated from the skin into the lowest adhesive layer migrates to the upper adhesive layers so that a deterioration of adhesion of the patch to the skin or skin irritation can be efficiently prevented.

Based on this type of structure, the present inventors studied effects of various penetration enhancers of the drug penetration into the skin and of physicochemical properties (e.g., hydrophobicity or hydrophilicity) of enhancers on the compatibility with other components of the adhesive layer into which they incorporated.

The above mentioned prior transdermal skin patch employs acrylic resin as an adhesive resin and controls water absorption capacity and solubility of drug on every adhesive layer by varying an amount of acrylic monomers. Therefore, most important requisites for a penetration enhancer for such multilayer laminate type of patch are an ability of constantly penetrating the drug cross the skin or every layer regardless the different content of acrylic monomer in every adhesive layer, and a good compatibility with the acrylic resin.

The present inventors selected various penetration enhancers from higher fatty acids, fatty alcohols, alcohols, glycols, surface active agents, fatty acid esters, glycerin, and fatty acid esters, and studied their penetration enhancing ability within the acrylic resin-type adhesives with a different content of acrylic monomer (and consequently different water absorption capacity) and their compatibility with the acrylic resin-type adhesives. As a result thereof, they found that hexylene glycol exhibits most effective penetration enhancing ability and good compatibility over a wide range of the content of the acrylic monomer. And, for the surface active-type penetration enhancers, hydrophobic surface active agents such as polyoxyethylene glycol ether or polyoxyethylene oleyl ether shows better penetration enhancing activity than hydrophilic agents.

### SUMMARY OF THE INVENTION

Thus, an object of the present invention is to provide a transdermal skin patch comprising a water-impermeable backing layer; a liner; and between the two, a multilayer laminated of from 2 to 5 adhesive layer, each of said adhesive layers contains a proper penetration enhancer showing a constant good penetration enhancing ability and a compatibility within acrylic adhesives with different water absorption capacities, as well as which is safe to the human skin.

According to the first aspect of the present invention, there is provided a transdermal skin patch to adhere to a patient's skin, which comprises a water-impermeable backing layer; a liner; and between the two, a multilayer laminated of from 2 to 5 adhesive layer, each of said adhesive layers comprising an oil-soluble drug, an adhesive resin, and a penetration enhancer, wherein each adhesive layer contains no water and has a different water absorption capacity in which the lowest layer which is to be contacted with the skin has the lowest water absorption capacity and the most upper layer in contact with the backing layer has the highest water absorption capacity, and said penetration enhancer being hexylene glycol present in an amount of 0.5 to 40% by weight.

### BRIEF DESCRIPTION OF DRAWING

This invention will be described in more detail with reference with accompanying drawings in which:

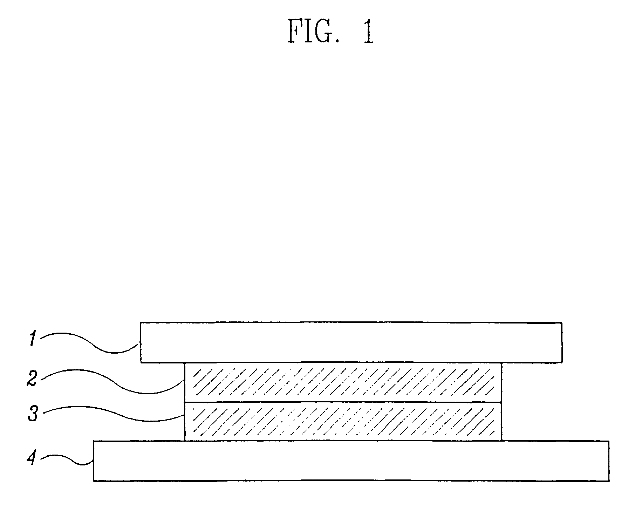

FIG. 1 is cross-sectional view of the patch according to present invention having a multilayer laminate structure including a backing layer (1), an upper adhesive layer (2), a lower adhesive layer (3) and a liner layer (4).

### DETAILED EXPLANATION OF THE INVENTION

When the patch according to the first aspect of the present invention is applied to the skin, the moisture evaporated from the skin is migrated into the lowest adhesive layer in contact with the skin, which comprises a water-absorptive material. Then, the moisture migrates in the lowest layer to upper layers so that the solubility of the drug in the upper layer is reduced, while the drug in the lowest layer is migrated into the skin. This promotes a migration of the drug from the upper layer to the lower layer, thereby the lowest layer in contact with the skin always can release a constant amount of drug. And, a skin irritation and deterioration of adherence of the patch due to accumulation of the sweat or secretions from the skin can be effectively prevented.

According to the other aspect of the present invention, if a penetration of a large amount of the drug at the early time of application thereof to the skin is desired ("Burst effect"), a transdermal skin patch comprising a water-permeable backing layer; a liner; and between the two, a multilayer laminated of from 2 to 5 adhesive layer, each of said adhesive layers comprising an oil-soluble drug, an adhesive resin, and a penetration enhancer, wherein each adhesive layer contains no water and has a different water absorption capacity in which the lowest layer which is to be contacted with the skin has the highest water absorption capacity and the most upper layer in contact with the backing layer has the lowest water absorption capacity, and said penetration enhancer being hexylene glycol present in an amount of 0.5 to 40% by weight is provided.

When the patch according to the second aspect of the present invention is applied to the skin, the moisture evaporated from the skin is prevented from being migrated into the upper adhesive layer. Thus, although a large amount of the drug or active ingredient can be penetrated cross the skin just after adhering it to the skin by selecting an adhesive layer with a desired water absorptive capacity and incorporating the drug thereinto to a saturation, when moisture is evaporated from the skin into the lowest adhesive layer, the solubility of the drug becomes reduced until the moisture evaporation reaches a steady state. At a steady state, a constant amount of drug is migrated into the skin.

The hexylene glycol selected as a penetration enhancer according to the present invention exhibits excellent enhancing effect of penetration of the drug within various acrylic resin-type adhesive layer as well as shows good compatibility with the acrylic resin, as explained below.

The adhesive layer of the patch according to the present invention may further include polyoxyethylene lauryl ether or hydrophobic surface active agents in order to obtain a synergism in improving the penetration of the drug. The hydrophobic surface active agents may include, no limited thereto, N,N-dihydroxyethyl lauramide, polyoxyethylene oleyl ether, polyoxyethylene lauryl ether, glycol monolaurate, propylene glycol monolaurate, and sorbitan monolaurate. Polyoxyethylene lauryl ether wherein the mole number of ethylene oxide is from 2 to 10 or polyoxyethylene oleyl ether wherein the mole number of ethylene oxide is from 2 to 7 is preferred.

The drug or active ingredient incorporated into the adhesive layers according to the present invention may include antipyretics, analgesics and antiinflammatory agents such as ibuprofenn, indomethacin, ketoprofen, flurbiprofen, piroxicam, ketorolac, diclofenac, mefenamic acid, salicylic acid, or pharmaceutically acceptable salts or derivatives thereof; adrenal cortical hormones such as dexamethasone, hydrocortisone, prednisolone, betamethasone, triamcinolone acetonide, fluocinolone acetonide, or pharmaceutically acceptable salts or derivatives thereof; beta-blockers such as propranolol, atenolol, pindolol, timolol, bupranolol, metoprolol, alprenolol, oxprenolol, or phamrceutically acceptable salt or derivatives thereof; calcium antagonists such as mifedipine, verpamil, diltiazem, or pharmaceutically acceptable salts or derivatives thereof;other cardiovascular agents such as clonidine, enalapril, prazosin, nitroglycerin, isosorbide dinitrate; female sex hormones such as estrrradiol, ethinylestradiol, progesterone, or pharmaceutically acceptable salts or derivatives thereof; male sex hormones such as testosterone or pharmaceutically acceptable salts or derivatives thereof; local anesthetics such as tetracaine, dibucaine, lidocaine, or pharmaceutically acceptable salts or derivatives thereof; antihistamines such as diphenhydramine, diphenhydramine maleate, chlorpheniramine, ketotifen, or pharmaceutically acceptable salts or derivatives thereof; synthetic narcotics such as buprenorphine, fentanyl, sufentanil, or pharmaceutically acceptable salts or derivatives thereof; bronchodilators such as salbutamol or terbutaline; or smoking sessation aids such as nicotine.

The active ingredient is not limited to the exampls indicated above, and may be any cosmetically active agents. The amount of the active ingredients is not limited to a specific range, and may be determined by those skilled in the art depending on the purpose or use of the patch, for example it may vary between 1 and 40% by weight based on the total weight of the adhesive layer.

The multilayer laminate structure of the patch according to the present invention will be described hereinbelow.

The transdermal patch of the present invention consists of a backing layer; 2 - 5 adhesive layers; and a separable liner.

As the backing layer of the patch, any supporting materials which are typically used in the art may be employed. Examples of such supporting materials include polyethylene terephthalate, polyurethane, polyethylene, polypropylene, aluminized polyethylene, or the like, which may be in the form of single layer or multilayer laminate. In addition, their laminate together with a water-permeable materials such as a woven fabric, cotton fabric or non-woven fabric may be also used as the backing layer.

The adhesive layer of the invention containing an active ingredient or a drug, hexylene glycol as a penetration enhancer, and the like has a multilayer laminate structure of 2 to 5 layers. The thickness of each layer is typically 2 to 150µm, preferably 10 to 100µm. And the overall thickness of the laminated adhesive layer is typically 30 to 500µm, preferably 50 to 200µm.

As an adhesive resin for constituting the adhesive layer, acrylic resins are employed. Acrylic resin is advantageously employed, since the control of content of acrylic monomer in the acrylic resin is easy so that the adjustment of the water absorption capacity of the adhesive layer, or of the solubility of the drug in the adhesive layer.

Particularly, for the present invention, acrylic resins such as a copolymer of C₄-C₁₈ aliphatic alcohol with (meth)acrylic alkyl ester, or a copolymer of (meth)acrylic alkyl ester having C₄-C₁₈ alkyl group with other functional monomers are preferably employed as the adhesive resin.

Examples of the (meth)acrylic copolymer may include butyl acrylate, isobutyl acrylate, hexyl acrylate, octyl acrylate, 2-ethylhexyl acrylate, isooctyl acrylate, decyl acrylate, isodecyl acrylate, lauryl acrylate, stearyl acrylate, methyl methacrylate, ethyl methacrylate, butyl methacrylate, isobutyl methacrylate, 2-ethylhexyl methacrylate, isooctyl methacrylate, decyl methacrylate, etc. Examples of other functional monomers may include a monomer containing hydroxy group, a monomer containing carboxyl group, a monomer containing amide group, a monomer containing amino group. Examples of the monomer containing hydroxy group may include hydroxy alkyl (meth)acrylate such as 2-hydroxyethyl (meth) acrylate, and hydroxypropyl (meth) acrylate.

Examples of the monomer containing carboxyl group may include α-β unsaturated carboxylic acid such as acrylic acid, and methacrylic acid; maleic mono alkyl ester such as butyl maleate and the like; maleic acid; fumaric acid; crotonic acid and the like; and anhydrous maleic acid. Examples of the monomer containing amide group may include alkyl (meth)acrylamide such as acrylamide, dimethyl acrylamide, and diethyl acrylamide alkylethylmethylol (meth) acrylamide such as butoxymethyl acrylamide, and ethoxymethyl acrylamide; diacetone acrylamide; vinyl pyrrolidone; dimethyl aminoacrylate.

In addition to the above exemplified monomers, vinyl acetate, styrene, α-methylstyrene, vinyl chloride, acrylonitrile, ethylene, propylene, butadiene and the like may be employed.

When an acrylic resin is employed as the adhesive resin for the adhesive layer, the adhesive layer may contain more than 50% of acrylic resin in the term of (meth)acrylic alkyl ester.

Further, for the purpose of increasing or decreasing the water absorption capacity of the adhesive layers, the acrylic resin may be copolymerized with a hydrophilic monomer, a carboxyl group-containing monomer, a amide group-containing monomer and an amino group-containing monomer.

Alternatively, the water absorption capacity of the adhesive layer can be also regulated by incorporating therein highly water-absorptive resins, polyols and water-absorptive inorganic materials. Examples of the highly water-absorptive resins may include a mucopolysaccharide such as hyaluronic acid, chondroitin sulfate, dermatan sulfate and the like; polymers having a large number of hydrophilic groups in the molecule such as chitin, chitin derivatives, starch and carboxy-methylcellulose; and semi-synthetic and synthetic highly water-absorptive resin such as polymers of polyacrylic, polyoxyethylene, polyvinyl alcohol and polyacrylonitrile. Examples of the water-absorptive inorganic materials incorporated into the adhesive layer for the purpose of regulating the layer's water absorptive capacity, may include powdered silica, zeolite, and powdered ceramics. Examples of the polyols may include propylene glycol, glycerin, and sorbitol.

These substances for regulating the water-absorption capacity of the adhesive layer may be employed in an amount of 0.1-40% by weight, preferably 1-20% by weight.

The adhesive layers may contain a tackifying agent and as a tackifying agent, there may be included, but not intended to be limited thereto, rosin resin, polyterphene resin, petroleum resin, terpene phenol resin.

The adhesive layers may contain penetration enhancer, for example dodecyl sulfoxide mono- or dimethyl acetamide, N-hydroxy ethyl lactide, higher fatty acid esters, salicylic acid, sorbitol, urea, glycerin, squalene, squalane, acetylated lanolin, cetyl laurate, olive oil, castor oil, lauric acid, oleic acid, lauryl alcohol, oleyl alcohol, ethoxystearyl alcohol, liquid paraffin, vaseline, camphor, glycerin fatty acid ester, fatty acid mono(or di-) ethanol amide, ethylene glycol mono ethyl ether, polyoxyethylene alkyl ether, polyoxyethylene alkyl ester, polyoxypropylene alkyl ether, propylene glycol mono(di)alkyl ester, propylene glycol monolaurate, polyoxyethylene lauryl ether, and pyrrolidone derivatives. The amount of the penetration enhancer is 0.1 to 40% by weight, preferably 1 to 20% by weight.

The adhesive layer may be incorporated with various additives, for example a tackifying agent such as rosin, polyterphene, cumaroindene, petroleum, or terphen phenol resins, a plasticizer such as liquid polybutene, mineral oils, lanoline, liquid polyisoprene, liquid polyacrylate; a filler; and an antioxidant.

Further, in order to improve tackiness of the adhesive layer of the patch according to the present invention, it may contain powdery organic polymers such as cellulose, polyethylene, nylon 6, nylon 12, polypropylene, polyethylene terephthalate, alginic acid, hyaluronic acid, chitin and their derivatives, and/or inorganic powders such as zinc oxide, calcium oxide, silica, kaolin, talc or titanium oxide. These materials may be present in an amount of 0.1-30% by weight, preferably 0.5-10% by weight based on the total amount of the adhesive layer.

### PREFERRED EMBODIMENT OF THE INVENTION

The present invention will be embodied by way of the following examples. However, these examples are provided for the illustration purpose only and should not be construed as limiting the scope of the invention, which is properly delineated in the accompanying claims. The parts or percents in Examples are based on the weight, unless indicated otherwise.

### Reference Example 1

### (A) Preparation of adhesive resin (1)

To a reaction vessel equipped with a reflux condenser and a stirrer, 97.4 parts of 2-ethylhexyl acrylate, 2.5 parts of methacrylic acid, 0.1 parts of polyethylene glycol di-methacrylate, 1.0 parts of benzoyl peroxide and 100 parts of ethyl acetate were added and, under nitrogen atmosphere, the polymerization reaction was carried out under stirring at the temperature of 60°C.

In order to regulate the polymerization degree, 100 parts of ethyl acetate was added to the reaction mixture gradually during the polymerization reaction.

The reaction was completed 9 hours after, when the polymerization degree was 99.9%. To the resulting polymer solution, ethyl acetate was added to adjust the solid content to about 40% by weight. Finally, there was obtained a copolymer of ethylhexyl acrylate, methacrylic acid and polyethylene glycol dimethacrylate, which will be employed as an adhesive resin.

### (B) Preparation of adhesive resin (2)

Under the same conditions as described above, the copolymerization reaction was carried out by employing 70 parts of 2-ethylhexyl acrylate, 10 parts of acrylic acid, 1.0 parts of benzoyl peroxide (BPO) and 20 parts of vinyl acetate. The polymerization degree was more than 99.9%. Aluminum acetate was added to the polymerization product under stirring (200rpm) to obtain a self-curable product. To the resulting polymer solution, ethyl acetate was added to adjust the solid content to about 40% by weight.

Finally, there was obtained a copolymer of ethylhexyl acrylate, methacrylic acid and vinyl acetate, which will be employed as an adhesive resin.

### Reference Example 2:

### Preparation of lower adhesive layer

To a mixture of the adhesive resins (1) and (2) obtained in the above, and the ingredients shown in Table 1 and 2, ketoprofen in an amount of 15% by weight based on the solid content of the mixture was dissolved in the mixture. The resulting mixture was coated onto a silicone-treated PET separate liner. At this time, the amount of the mixture was adjusted in such a way that the coating thickness after drying become 50µm.

**Table 1 :**

| Composition of lower adhesive layer | | | | | | | | |
|---|---|---|---|---|---|---|---|---|
| No. | Adhesive resin 1 | Cellulose * | Silica ** | HG | PG | POEL | SML | Water Absorption Capacity *** |
| 1 | 85 | | | | | | | 1.5 |
| 2 | 75 | | | 10 | | | | 1.9 |
| 3 | 75 | | | | 10 | | | 1.9 |
| 4 | 75 | | | | | 10 | | 1.9 |
| 5 | 75 | | | | | | 10 | 1.9 |
| 6 | 70 | | | 10 | 5 | | | 2.1 |
| 7 | 70 | | | 10 | | 5 | | 2.1 |
| 8 | 70 | | | 10 | | | 5 | 2.1 |
| 9 | 70 | | | | 10 | 5 | | 2.1 |
| 10 | 70 | | | | | 10 | 5 | 2.0 |
| 11 | 72 | 3 | | 10 | | | | 2.5 |
| 12 | 72 | 3 | | | 10 | | | 2.5 |
| 13 | 72 | 3 | | | | 10 | | 2.5 |
| 14 | 72 | 3 | | | | | 10 | 2.5 |
| 15 | 69 | 3 | 3 | 10 | | | | 3.1 |
| 16 | 69 | 3 | 3 | | 10 | | | 3.1 |
| 17 | 69 | 3 | 3 | | | 10 | | 3.1 |
| 18 | 69 | 3 | 3 | | | | 10 | 3.1 |

| | | | | | | | | |
|---|---|---|---|---|---|---|---|---|
| * Cellulose having a diameter of 5 - 15 µm. | | | | | | | | |
| ** Silica having a diameter of 3 - 5 µm. HG = hexylene glycol PG = Propylene glycol POEL = Polyoxyethylene lauryl ether (E.O. = 9 moles) SML = Sorbitan monolaurate | | | | | | | | |
| *** Quantitative determination of the water content | | | | | | | | |

A piece (5 x 5 cm² ) cut out from each adhesive layers, which are coated into a polyethylene film to a thickness of 100µm, was dipped into a purified water for 1 minute, and was subjected to chromatography to quantitatively determine the water content in the adhesive layers.

**Table 2 :**

| Composition of upper adhesive layer | | | | | | |
|---|---|---|---|---|---|---|
| No. | Adhesive resin 2 | HG | PG | POEL | SML | Water Absorption Capacity |
| 19 | 85 | | | | | 3.2 |
| 20 | 75 | 10 | | | | 3.6 |
| 21 | 75 | | 10 | | | 3.6 |
| 22 | 75 | | | 10 | | 3.6 |
| 23 | 75 | | | | 10 | 3.6 |
| 24 | 70 | 10 | 5 | | | 3.8 |
| 25 | 70 | 10 | | 5 | | 3.8 |
| 26 | 70 | 10 | | | 5 | 3.8 |
| 27 | 70 | | 10 | 5 | | 3.8 |
| 28 | 70 | | | 10 | 5 | 3.7 |

### Example 1 : Fabrication of patches 1 - 18

The upper adhesive layer(2) as shown in Table 2 was adhered by rolling onto a backing layer (polyethylene film) and then, the lower adhesive layer(3) as shown in Table 1 was laminated thereonto to produce a transdermal patch having two adhesive layer laminate structure as shown in Table 3.

### Example 2 : Fabrication of patch

By follwoing the procedure in Example 1 and employing the adhesive layer No. 7 in Table 1 as a lower layer, the adhesive layer No. 25 in Table 2 as a upper layer, and ketorolac as an active ingredient, there is obtained a patch containing ketorolac and having two adhesive layer laminate structure.

### Example 3 : Fabrication of patche

By follwoing the procedure in Example 1 and employing the adhesive layer No. 8 in Table 1 as a lower layer, the adhesive layer No. 26 in Table 2 as a upper layer, and estradiol as an active ingredient, there is obtained a patch containing estradiol and having two adhesive layer laminate structure.

### Experimental Example 1 : Percutaneous penetration test

Using a male guinea pig weighing about 350g, the abdominal hair was removed using a hair clipper Then, a part of the abdominal skin was excised, stored in a refrigerator (below -20°C) and used after thawing at needs.

The excised skin was placed in the middle of the-Franz-type diffusion cell with its corneous side looking upward and the space below the cell was charged with 0.05M phosphate buffered saline(pH 7.4).

The adhesives shown in Table 1 below were applied on the skin while the buffer solution(receiver solution) was stirred at a constant speed of 600rpm.

At varying intervals, a portion of the receiver solution was taken for the test and a fresh buffer solution was supplemented in the same amount as taken therefrom. From the test solution, the concentration of ketoprofen was determined by High Pressure Liquid Chromatography(HPLC).

### [Conditions for HPLC]

. Column : C₁₈µ Bondapak [Waters Chromatography,
   Inc., milton Massachusetts 01757 USA]
. Mobile phase : 55:45 V/V
   Mixture of methanol : 0.02M phosphate buffered solution(pH 4.0)
. Flow rate : 1 ml/min.
. Detector : 254nm wave length ultraviolet

The results are shown in Table 3 below.

### Experimental Example 2 : Skin primary irritation test (Draize's grade method)

An adhesive prepared in the same manner as described in Example 1 was cut to the size of 2.5 cm² and after the separate liner was removed therefrom, the adhesive was applied on the forearm of healthy volunteer for 24 hours. 24 hours later, the adhesive was removed and, after 30 minutes, the level of the skin primary irritation was observed and estimated according to the Draize' grade method.

| Score Range | Evaluation |
|---|---|
| 0.0 - 0.09 | No irritation |
| 0.1 - 0.50 | Minimum irritation |
| 0.51 - 1.50 | Very slight irritation |
| 1.51 - 3.00 | Slight irritation |
| 3.01 - 5.00 | Irritation |
| 5.01 - 6.50 | Severe irritation |
| 6.51 - 8.00 | Extremely severe irritation |

**Table 3 :**

| Patches used for the percutaneous penetration test | | | | | |
|---|---|---|---|---|---|
| Patch No. | Upper layer + Lower layer | (1) Skin across migration rate*1 (µg/cm² /hr) | Retention Time (hr) | (2) Primary Irritation | No. of animal (1) --(2) |
| 1 | 1 + 19 | 17.5±2.3 | 2.19±0.31 | 0.48 | 5 / 10 |
| 2 | 2 + 20 | 34.2±3.9 | 1.89±0.46 | 0.82 | 6 / 10 |
| 3 | 3 + 21 | 33.8±4.2 | 1.92±0.43 | 0.63 | 6 / 10 |
| 4 | 4 + 22 | 28.9±2.4 | 1.88±0.55 | 0.75 | 6 / 10 |
| 5 | 5 + 23 | 29.3±3.3 | 1.90±0.51 | 0.70 | 6 / 10 |
| 6 | 6 + 24 | 42.6±4.2 | 1.79±0.34 | 1.32 | 6 / 10 |
| 7 | 7 + 25 | 45.2±3.8 | 1.84±0.57 | 1.45 | 6 / 10 |
| 8 | 8 + 26 | 44.8±1.9 | 1.77±0.73 | 1.25 | 6 / 10 |
| 9 | 9 + 27 | 38.7±3.0 | 1.82±0.45 | 1.07 | 6 / 10 |
| 10 | 10 + 28 | 36.2±2.5 | 1.84±0.67 | 1.39 | 6 / 10 |
| 11 | 11 + 20 | 32.2±1.7 | 1.72±0.47 | 0.72 | 5 / 10 |
| 12 | 12 + 21 | 24.2±2.5 | 1.65±0.25 | 0.51 | 5 / 10 |
| 13 | 13 + 22 | 21.2±3.2 | 1.69+0.36 | 0.46 | 5 / 10 |
| 14 | 14 + 23 | 20.5±2.4 | 1.71±0.70 | 0.61 | 5 / 10 |
| 15 | 15 + 20 | 30.8±0.9 | 1.57±0.52 | 0.88 | 5 / 10 |
| 16 | 16 + 21 | 20.4±3.7 | 1.60±0.63 | 0.57 | 5 / 10 |
| 17 | 17 + 22 | 19.1±1.8 | 1.54±0.37 | 0.48 | 5 / 10 |
| 18 | 18 + 23 | 18.2±2.1 | 1.59±0.74 | 0.43 | 5 / 10 |

| | | | | | |
|---|---|---|---|---|---|
| Note) *1 : Skin across migration rate 24 hours after Applying the patch. | | | | | |

The results in Table 3 proves that hexylene glycol is a more appropriate penetration enhancer for the multilayer laminate structure of the patch than propylene glycol, a most widely used penetration enhancer.

Further, it can be seen that combinations of hexylene glycol and other penetration enhancer show a synergic effect in the migration of drug through skin and are safe to the living body.

Moreover, hexylene glycol exhibits a constant effect to enhance the penetration of drug even though the water retention of the lower adhesive layer is changed. By contrary, propylene glycol exhibits a reduced effect to enhance the penetration of drug when the water retention of the lower adhesive layer is changed.

## Claims

1. A transdermal skin patch to adhere to a patient's skin, which comprises a water-impermeable backing layer; a liner; and between the two, a multilayer laminated of from 2 to 5 adhesive layer, each of said adhesive layers comprising an active ingredient, an adhesive resin, and a penetration enhancer, wherein each adhesive layer contains no water and has a different water absorption capacity in which the lowest layer which is to be contacted with the skin has the lowest water absorption capacity and the most upper layer in contact with the backing layer has the highest water absorption capacity, and said penetration enhancer being hexylene glycol present in an amount of 0.5 to 40% by weight.

2. The patch of claim 1, wherein said adhesive layer further comprises one selected from a group consisting of N,N-dihydroxyethyl lauramide, polyoxyethylene oleyl ether, polyoxyethylene lauryl ether, glycol monolaurate, propylene glycol monolaurate, and sorbitan monolaurate, as an additional penetration enhancer.

3. The patch of claim 1 or 2, wherein said active ingredient is one selected from a group consisting of ketoprofen, diclofenac, indomethacin, flurbiprofen, piroxicam and ketorolac.

4. The patch of claim 1 or 2, wherein said active ingredient is one selected from a group consisting of estradiol, prednisolone, nifedipine, buprenorphine, progesterone, ketotifenn, testosterone, isosorbide dinitrate, nitroglycerin and nicotine.

5. A transdermal skin patch to adhere to a patient's skin, which comprises a water-permeable backing layer; a liner; and between the two, a multilayer laminated of from 2 to 5 adhesive layer, each of said adhesive layers comprising an active ingredient, an adhesive resin, and a penetration enhancer, wherein each adhesive layer contains no water and has a different water absorption capacity in which the lowest layer which is to be contacted with the skin has the highest water absorption capacity and the most upper layer in contact with the backing layer has the lowest water absorption capacity, and said penetration enhancer being hexylene glycol present in an amount of 0.5 to 40% by weight

## Patentansprüche

1. Transdermales Hautpflaster zum Haften an der Haut eines Patienten mit
einer wasserundurchlässigen Deckschicht, einer Einlage und einer Mehrfachschicht dazwischen, die aus zwei bis fünf Haftschichten larniniert ist, wobei
jede der Haftschichten ein aktives Ingredienz, ein Klebharz und einen Penetrationsverstärker aufweist,
jede Haftschicht wasserfrei ist und ein unterschiedliches Wasserabsorptionsvermögen besitzt, wobei die unterste Schicht, die mit der Haut in Kontakt kommen soll, das niedrigste Wasserabsorptionsvermögen besitzt und die oberste Schicht in Kontakt mit der Deckschicht das höchste Wasserabsorptionsvermögen besitzt, und
der Penetrationsverstärker Hexylenglykol ist, das in einer Menge von 0.5 bis 40 Gewichts-% vorhanden ist.

2. Pflaster nach Anspruch 1, bei dem die Haftschicht als einen zusätzlichen Penetrationsverstärker weiter ein Mittel einer Gruppe aufweist, die aus N,N-Dihydroxyethyllauramid, Polyoxyethylenoleylether, Polyoxyethylenlaurylether, Glykolmonolaurat, Propylenglykolmonolaurat und Sorbitanmonolaurat besteht,

3. Pflaster nach Anspruch 1 oder 2, bei dern das aktive Ingredienz ein Mittel ist, das aus einer Gruppe ausgewählt ist, die Ketoprofen, Diclofenac, Indometacin, Flurbiprofen, Piroxicarn und Ketorolac enthält.

4. Pflaster nach Anspruch 1 oder 2, bei dem das aktive Ingredienz ein Mittel ist, das aus einer Gruppe ausgewählt ist, die aus Estradiol, Prednisolon, Nifedipin, Buprenorphin, Progesteron, Ketotifen, Testosteron, Isosorbiddinitrat, Nitroglycerin und Nikotin besteht.

5. Transdermales Hautpflaster zum Haften an der Haut eines Patienten, mit
einer wasserdurchlässigen Deckschicht, einer Einlage, und einer Mehrfachschicht dazwischen, die aus zwei bis fünf Haftschichten laminiert ist, wobei
jede der Haftschichten ein aktives Ingredienz, ein Klebharz und einen Penetrationsverstärker aufweist,
jede Haftschicht wasserfrei ist und ein unterschiedliches Wasserabsorptionsvermögen besitzt, wobei die unterste Schicht, die mit der Haut in Kontakt kommen soll, das höchste Wasserabsorptionsvermögen besitzt, und die oberste Schicht in Kontakt mit der Deckschicht das niedrigste Wasserabsorptionsvermögen besitzt, und
der Penetrationsverstärker Hexylenglykol ist, das in einer Menge von 0.5 bis 40 Gewichts-% vorhanden ist.

## Revendications

1. Timbre cutané transdermique destiné à adhérer à la peau d'un patient, qui comprend une couche dorsale imperméable à l'eau, un revêtement et, entre les deux, une multicouche stratifiée de 2 à 5 couches adhésives, chacune desdites couches adhésives comprenant un ingrédient actif, une résine adhésive et un agent augmentant la pénétration, où chaque couche adhésive ne contient pas d'eau et a une capacité d'absorption d'eau différente, où la couche inférieure qui doit être mise en contact avec la peau a la plus basse capacité d'absorption d'eau et la couche supérieure en contact avec la couche dorsale a la plus haute capacité d'absorption d'eau, et ledit agent augmentant la pénétration est l'hexylèneglycol présent en une quantité de 0,5 à 40 % en masse.

2. Timbre selon la revendication 1, où ladite couche adhésive comprend en outre une substance choisie dans un groupe consistant en le N,N-dihydroxyéthyllauramide, le polyoxyéthylèneoléyléther, le polyoxyéthylènelauryléther, le monolaurate de glycol, le monolaurate de propylèneglycol et le monolaurate de sorbitan, comme agent augmentant la pénétration supplémentaire.

3. Timbre selon la revendication 1 ou 2, où ledit ingrédient actif est un ingrédient actif choisi dans un groupe consistant en le ketoprofène, le diclofénac, l'indométhacine, le flurbiprofene, le piroxicame et le ketorolac.

4. Timbre selon la revendication 1 ou 2, où ledit ingrédient actif est un ingrédient actif choisi dans un groupe consistant en l'estradiol, la prédnisolone, la nifédipine, la buprénorphine, la progestérone, le kétotifène, la testostérone, le dinitrate d'isosorbide, la nitroglycérine et la nicotine.

5. Timbre cutané transdermique destiné à adhérer à la peau d'un patient, qui comprend une couche dorsale perméable à l'eau, un revêtement et, entre les deux, une multicouche stratifiée de 2 à 5 couches adhésives, chacune desdites couches adhésives comprenant un ingrédient actif, une résine adhésive et un agent augmentant la pénétration, où chaque couche adhésive ne contient pas d'eau et a une capacité d'absorption d'eau différente, où la couche inférieure qui doit être mise en contact avec la peau a la plus haute capacité d'absorption d'eau et la couche supérieure en contact avec la couche dorsale a la plus basse capacité d'absorption d'eau, et ledit agent augmentant la pénétration est l'hexylèneglycol présent en une quantité de 0,5 à 40 % en masse.
